**Europäisches Patentamt**

**European Patent Office**

**Office européen des brevets**

(19)

(11) Veröffentlichungsnummer : **0 162 362 B1**

(12) # EUROPÄISCHE PATENTSCHRIFT

(45) Veröffentlichungstag der Patentschrift :
11.11.87

(21) Anmeldenummer : 85105471.8

(22) Anmeldetag : 04.05.85

(51) Int. Cl.⁴ : **C 07 C 29/04**, C 07 C 31/10, C 07 C 31/12

(54) **Verfahren zur kontinuierlichen Herstellung von Alkoholen.**

(30) Priorität : 24.05.84 DE 3419392

(43) Veröffentlichungstag der Anmeldung :
27.11.85 Patentblatt 85/48

(45) Bekanntmachung des Hinweises auf die Patenterteilung : 11.11.87 Patentblatt 87/46

(84) Benannte Vertragsstaaten :
AT BE DE FR GB IT NL SE

(56) Entgegenhaltungen :
DE-A- 2 759 237
DE-B- 1 065 398
US-A- 2 050 445
US-A- 2 533 808
US-A- 4 352 945

(73) Patentinhaber : DEUTSCHE TEXACO AKTIENGESELL-SCHAFT
Überseering 40
D-2000 Hamburg 60 (DE)

(72) Erfinder : Neier, Wilhelm, Dr.
An der Landwehr 40
D-4134 Rheinberg 3 (DE)
Erfinder : Webers, Werner
Rektor-Horn-Strasse 42
D-4134 Rheinberg 3 (DE)
Erfinder : Dettmer, Michael, Dr.
Am Alten Kirchweg 16
D-2056 Glinde (DE)
Erfinder : Osterburg, Günther
Buchenstrasse 1
D-4100 Duisburg 17 (DE)

(74) Vertreter : Schupfner, Gerhard D.
Müller, Schupfner & Gauger Karlstrasse 5 Postfach 14 27
D-2110 Buchholz/Nordheide (DE)

## Beschreibung

Die vorliegende Erfindung betrifft ein Verfahren zur kontinuierlichen Herstellung von Isopropylalkohol und sec-Butylalkohol durch direkte katalytische Hydratisierung der entsprechenden Olefine mit Wasser in Gegenwart saurer Katalysatoren in einem Riesel- oder Sumpfreaktor bei erhöhter Temperatur and unter erhöhtem Druck und Rückführung des bei der Umsetzung als Nebenprodukt gebildeten und aus dem Umsetzungsprodukt isolierten Ethers.

Es ist bekannt, niedere sekundäre Alkohole dadurch herzustellen, daß man die entsprechenden Olefine zusammen mit Wasser bei erhöhtem Druck und erhöhter Temperatur an sauren Katalysatoren umsetzt. Als Katalysatoren kommen im wesentlichen organische Sulfonsäureharze sowie anorganische, mit Säuren beladene poröse Trägermaterialien in Betracht.

Die Selektivität dieser Verfahren wird durch die Bildung von Ethern, siehe z. B. Hydrocarbon Processing, Nov. 1972, S. 113-116, verschlechtert. Es ist auch bekannt, daß bei einem kontinuierlichen Prozeß die Bildung des Ethers durch Rückführung in den Einsatzstrom aufgrund der Gleichgewichtslage unterdrückt werden kann, siehe z. B. Chemical Engineering, September 4, 1972, S. 50, 51 oder die DE-A 2 759 237.

In der DE-B-1 065 398 ist ein Verfahren zur Herstellung von Isopropylalkohol mit wäßriger Schwefelsäure beschrieben, wobei der Äther separat von den Reaktanden in den Reaktor eingespeist wird.

Alternative Möglichkeiten zur Erhöhung der Gesamtselektivität eines kontinuierlichen Prozesses bestehen durch separate Spaltung des gebildeten Ethers in entweder Ausgangsolefin und Alkohol, siehe z. B. die US-Patentschrift 4 352 945 oder in zwei Moleküle Alkohol pro Mol Ether in Gegenwart eines Wasserüberschusses, siehe die ältere deutsche Patentanmeldung P 33 36 644.

Die beiden letztgenannten Möglichkeiten haben u. a. den Nachteil, daß zu ihrer Realisierung ein weiterer Reaktor benötigt wird. Bei der erstgenannten Möglichkeit, der Etherrückführung in den Eingangsstrom, ergibt sich — wie eigene Versuche zeigen (vgl. Vergleichsbeispiele 3, 4 und 6) — ein starker Abfall der Reaktorleistung.

Der vorliegenden Erfindung liegt die Aufgabe zugrunde, die Selektivität der Alkoholbildung zu erhöhen, ohne daß dabei durch Unterdrückung der Etherbildung Leistungseinbußen oder ein größerer apparativer Aufwand durch weitere Reaktoren in Kauf genommen werden müssen.

Die Aufgabe wird erfindungsgemäß dadurch gelöst, daß der Ether separat von den Reaktanden 5 bis 30 % von Ende der Reaktionsstrecke, bezogen auf die Gesamtlänge, in den Reaktor eingespeist wird.

Gemäß einer bevorzugten Ausführungsfrom wird der Ether 10 bis 20 % von Ende der Reaktionsstrecke eingespeist.

Überraschenderweise wurde nämlich gefunden, daß sich die Reaktorleistung sowohl bei Verfahren mit einem Riesel — als auch bei solchen mit einem Sumpfreaktor gegenüber der herkömmlichen Verfahrensweise mit Etherrückführung steigern läßt, wenn man das rückgeführte Nebenprodukt nicht in den Einsatzstrom gibt, sondern es erst kurz vor Ende der Reaktionsstrecke zugibt. Trotz einer sehr kurzen Reaktionsstrecke von nur bis zu 5 bis 10 % der Gesamtlänge wird eine quantitative Ätherspaltung erreicht. Dies ist für den Fachmann besonders überraschend.

Die beigefügten Zeichnungen erläutern Ausführungsbeispiele des erfindungsgemäßen Verfahrens.

Fig. 1 zeit das Schema eines Rieselreaktors für die Hydratisierung von Olefinen mit gezielter Etherrückführung ;

Fig. 2 zeigt das Schema eines Sumpfreaktors für die Hydratisierung von Olefinen mit gezielter Etherrückführung ;

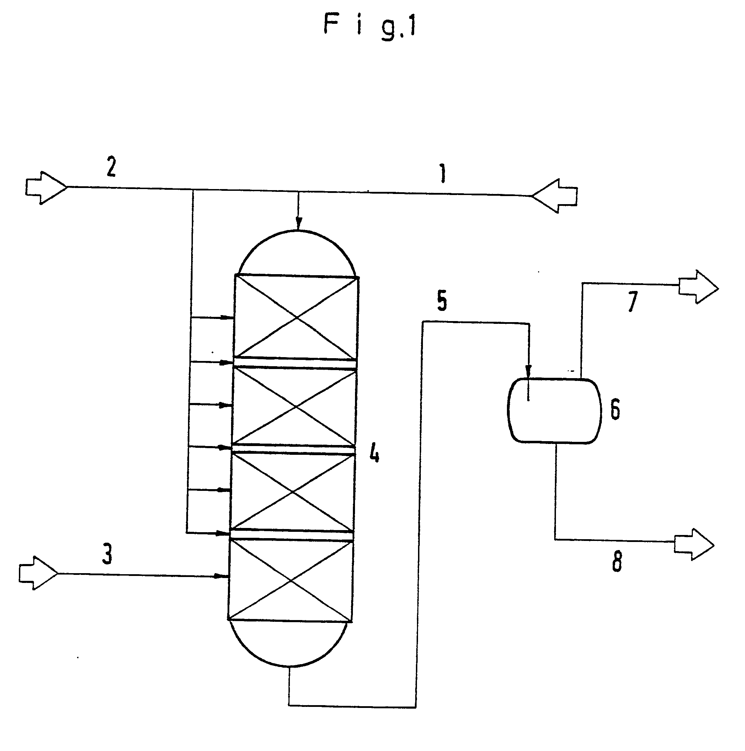

Gemäß Fig. 1 wird über Leitung 1 ein Olefin/Alkan-Gemisch und über Leitung 2 das ·Prozeßwasser in einen mit einem sauren Katalysator gefüllten Rohrreaktor 4 dosiert. Zwecks besserer Beherrschung der Relationswärme kann die Wasserzugabe auch wie dargestellt abschnittsweise erfolgen. In einer säurekatalysierten Mehrphasenreaktion bei erhöhtem Druck und erhöhter Temperatur bilded sich dann der gewünschte Alkohol. Die unerwünschte Neben- bzw. Folgereaktion zum Ether wird durch Zudosierung des vom Produkt abgetrennten, rückgeführten Ethers unterdrückt. Die Zudosierung erfolgt dabei in den stromabwärts gelegenen Reaktorteil über Leitung 3.

Das Reaktionsprodukt wird über Leitung 5 in einen Abscheider 6 geführt und dort in eine organische Phase, Leitung 7, und eine wäßrige Phase, Leitung 8, getrennt. Die Aufarbeitung der beiden Phasen geschieht in bekannter Weise. Das abgetrennte Olefin der organischen Phase kann je nach gewünschtem Umsatzgrad zurückgeführt werden. Der abgetrennte Ether wird über Leitung 3 in den Prozeß zurückgeführt. Die spezifische Reaktorleistung, gebildeter Alkohol pro Katalysatorvolumen und Zeit, ist gleich oder größer der Leistung eines entsprechenden Reaktors ohne Ethereinspeisung. Sie ist größer als die eines Reaktors mit Etherrückführung über Leitung 1.

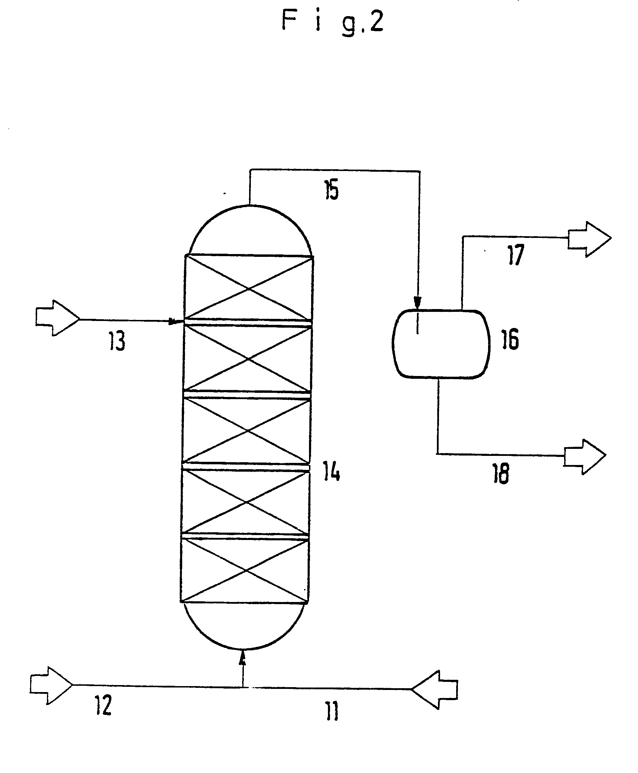

In Fig. 2 ist ein Anwendungsbeispiel für einen Sumpfreaktor 14 dargestellt. Über die Leitungen 11 und 12 werden das Olefin und das Prozeßwasser dem Reaktor zugeführt. Der vom Produktstrom abgetrennte Ether wird über Leitung 13 in den hinteren Teil des Reaktors rückgeführt. Die Trennung des Produktstromes in eine organische Phase, Strom 17, und eine wäßrige Phase, Strom 18, erfolgt in Abscheider 16. Die Aufarbeitung geschieht in üblicher Weise. Je nach Erfordernissen kann das abgetrennte Olefin nochmals umgesetzt werden. Bezüglich der Reaktorleistung gilt das für den Rieselreaktor Gesagte.

Als Katalysator wird ein für die Hydratisierung von Olefinen üblicher Katalysator eingesetzt. Besonders bevorzugt hierfür sind temperaturbeständige Ionenaustauscherharze vom Sulfonsäuretyp.

Die Reaktionstemperaturen liegen bei 100 bis 200 °C. Der Drusck liegt im Bereich von 40 bis 120 bar. Das Molverhältnis Mol Olefin zu Mol Wasser beträgt 0,5 : 1 bis 30 : 1. Bevorzugt wird zweiphasig gefahren unter Einsatz dampfförmigen Olefins und flüssigen Wassers.

Die im Kreis zu führende Menge an Ether liegt bezogen auf die Olefinmenge zwischen 5 und 30 Gew.%.

Die folgenden Beispiele erläutern die Erfindung, wobei auf die beigefügten Zeichnungen Bezug genommen wird.

Vergleichsbeispiel 1

In den Rieselreaktor 4 von 9 m Länge und 280 mm Durchmesser, der mit 450 l Amberlite 252, einem stark sauren Kationenaustauscherharz, gefüllt war, wurden stündlich 74,1 kg eines 92 %igen Propens über Leitung 1 und 540 kg entmineralisiertes Wasser über Leitung 2 eingespeist. Beide Ströme wurden über Vorwärmer auf Reaktionstemperatur gebracht und am Kopf dem Rieselreaktor 4 zugeführt. Zur Temperaturführung wurde ein Teil des Wasser vor dem Vorwärmer abgezweigt und an mehreren Stellen dem Reaktor zugeführt. Über Leitung 3 wurde in diesem Versuchslauf kein Ether in den Reaktor eingespeist.

Der Produktstrom 5 wurde in Abscheider 6 in eine wäßrige und eine organische Phase getrennt.

Über Leitung 7 wurden pro Stunde 29,4 kg einer organischen Phase mit folgender durchschnittlicher Zusammensetzung erhalten : 62,5 % Propan/Propen ; 17,0 % Diisopropylether (DIPE) ; 20,4 % Isopropylalkohol (IPA).

Über Leitung 8 wurden stündlich 583 kg wäßriges IPA erhalten, mit einem IPA-Gehalt von 11,5 % und einem DIPE-Gehalt von 0,1 %.

Der Reaktionsdruck lag bei 100 bar, die Reaktionstemperatur im Mittel bei 142 °C.

In diesem Versuchslauf wurden stündlich durchschnittlich 73,0 kg IPA und 5,6 kg DIPE erhalten. Die Katalysatorleistung lag bei 2,70 Mol/l Kat · h, die Etherbildung bei 7,1 % (IPA + DIPE = 100 %) und der Olefinumsatz bei ca. 80 %.

Vergleichsbeispiel 2

Vergleichsbeispiel 1 wurde mit der Maßgabe wiederholt, daß anstelle eines 92 % igen Propens jetzt die gleiche Menge eines 80 % igen Propens eingesetzt wurde. Alle anderen Bedingungen wurden konstant gehalten. Es wurden stündlich 54,1 kg IPA und 7,5 kg DIPE erhalten.

Die Katalysatorleistung lag bei 2,00 Mol IPA/l Kat · h, die Etherbildung bei 12,2 % (IPA + DIPE = 100 %).

Vergleichsbeispiel 3

In Anlehnung an US-PS 2 050 445 wurde das Vergleichsbeispiel 1 mit der Maßgabe wiederholt, daß unter sonst gleichen Reaktionsbedingungen dem Strom 1 jetzt 7,0 kg/h DIPE zugegeben wurden.

Der Propenumsatz sank auf 59 % ab. Es wurden 54,1 kg/h IPA und 9,5 kg/h DIPE erhalten.

Die Katalysatorleistung lag bei 2,00 Mol IPA/l Kat · h, die Etherbildung bei 4,4 %.

Vergleichsbeispiel 4

Das Vergleichsbeispiel 2 wurde mit der Maßgabe wiederholt, daß in den Einsatzstrom zusätzlich 7,0 kg/h DIPE eingespeist wurden. Alle anderen Bedingungen wurden konstant gehalten.

Es wurden stündlich 43,2 kg IPA und 10,1 kg DIPE erhalten. Die Katalysatorleistung lag bei 1,6 Mol IPA/l Kat · h, die Etherbildung noch bei 6,7 %.

Beispiel 1

Das Vergleichsbeispiel 3 wurde unter sonst gleichen Bedingungen wiederholt, nur daß die 7,0 kg/h DIPE erst 1 m vor Ende der 9 m langen Katalysatorstrecke eingespeist wurden.

Der Propenumsatz lag jetzt bei durchschnittlich 78 %. Es wurden 76,0 kg/h IPA und 7,0 kg/h DIPE erhalten.

Die Katalysatorleistung lag bei 2,81 Mol IPA/l Kat · h. Bei der Synthese insgesamt wurde kein Ether gebildet.

Beispiel 2

Das Beispiel 1 wurde mit der Maßgabe wiederholt, daß die eingespeiste Ethermenge auf 9 kg/h erhöht wurde.

Es wurden jetzt 76,9 kg/h IPA und 7,1 kg/h DIPE erhalten.

Die Katalysatorleistung lag jetzt bei 2,84 Mol IPA/l Kat · h. Es wurde zusätzlich DIPE zu IPA rückgespalten.

Beispiel 3

Das Vergleichsbeispiel 4 wurde mit der Maßgabe wiederholt, daß unter sonst gleichen Bedingungen die 7,0 kg Ether pro Stunde 1 Meter vom Ende der Katalysatorstrecke zugegeben wurde.

Es wurden stündlich 58,5 kg IPA und 7,6 kg DIPE erhalten.

Die Katalysatorleistung betrug 2,15 Mol IPA/l Kat · h. Die Etherbildung lag bei 1,0 %.

Beispiel 4

Beispiel 3 wurde mit der Maßgabe wiederholt, daß die Einspeisemenge für DIPE von 7,0 kg/h auf 9,0 kg/h angehoben wurde.

Es wurden wie im Beispiel 3 58,5 kg/h IPA und 7,6 kg/h DIPE erhalten. Es wurde zusätzlich Diisopropylether zu IPA gespalten.

### Vergleichsbeispiel 5

In den Sumpfreaktor 14 von 13,5 m Länge und 5 cm² freier Querschnittsfläche, der mit 6,75 l Amberlite 252, einem stark sauren Kationenaustauscherharz, gefüllt war, wurden über Leitung 12 stündlich 2 000 g Wasser und über Leitung 11 527 g eines 98,9 % n-Butene enthaltenden C₄-Schnittes sowie 8 270 g eines 90 %igen Buten-Recycle-Stromes zudosiert. Der Druck im Reaktor betrug 60 bar. Der mantelbeheizte Reaktor sowie der nicht gezeichnete Vorheizer wurden auf einer Temperatur von 155 °C gehalten. Der Produktstrom 15 wurde in dem Abscheider 16 in eine wäßrige und eine organische Phase getrennt.

Über Leitung 18 fielen pro Stunde 1 830 g einer wäßrigen Lösung an, die 1,1 % SBA enthielt. Die organische Phase wurde mittels einer kontinuierlichen Destillation aufgetrennt. Hierbei wurden pro Stunde 580 g sek.Butanol (SBA), 17 g Diisobutylether (DIBE) und 40 g Wasser von der C₄-Flüssiggasphase abgetrennt. An Flüssiggas fielen stündlich 8 330 g mit einem n-Buten-Gehalt von 90 % an. Ein Teil dieses Gases mußte wegen des Alkananteils des Einsatzgases ausgeschleust werden, die restlichen 8 270 g wurden, wie oben erwähnt, in den Reaktor über Leitung 11 zurückgeführt.

Insgesamt wurden stündlich 600 g SBA und 17 g DIBE erhalten. Der n-Buten-Umsatz lag bei 90 %. Die Reaktorleistung errechnete sich zu 1,20 Mol/l · h, der Ethergehalt (SBA + DIBE = 100 %) zu 2,8 %.

### Vergleichsbeispiel 6

Vergleichsbeispiel 5 wurde mit der Maßgabe wiederholt, daß dem C₄-Gasstrom in Leitung 11 stündlich 1 140 g DIBE zugegeben wurden. Zur Aufrechterhaltung eines konstanten Umsatzes mußte gleichzeitig der Frischgasstrom auf 189 g pro Stunde zurückgenommen werden.

Bei dieser Versuchseinstellung fielen insgesamt stündlich 495 g SBA und 900 g DIBE an. Es wurden also pro Stunde 240 g DIBE gespalten, wobei gleichzeitig ein Rückgang der Leistung auf 0,99 Mol/l h erfolgte.

### Beispiel 5

Vergleichsbeispiel 5 wurde mit der Maßgabe wiederholt, daß jetzt zusätzlich über Leitung 13 2,5 m vor dem Reaktorende stündlich 690 g DIBE eingespeist wurden. Gleichzeitig wurde, un den Butenumsatz zu SBA gleichzuhalten, die Einspeisung von frischem C₄-Gas auf 527 g zurückgenommen.

Mit der wäßrigen Phase aus Abscheider 16 fielen wieder stündlich 20 g SBA an. Aus der organischen Phase wurden neben 580 g SBA jetzt auch 690 g abgetrennt, so daß bei der Synthese insgesamt kein Ether gebildet wurde. Die Reaktorleistung lag wie bei Vergleichsbeispiel 5 bei 1,20 mol/l h.

### Beispiel 6

Beispiel 5 wurde mit der Maßgabe wiederholt, daß die über Leitung 13 zudosierte Menge DIBE auf 1 140 g pro Stunde erhöht wurde. Parallel dazu konnte die Frischgaseinspeisung auf stündlich 437 g zurückgenommen werden.

Der Alkoholanfall blieb gegenüber Beispiel 5 unverändert, an DIBE wurden stündlich 990 g abgetrennt.

Unter diesen Versuchsbedingungen wurden somit bei unveränderter Leistung stündlich 150 g DIBE zu Alkohol hydratisiert.

### Patentansprüche

1. Verfahren zur kontinuierlichen Herstellung von Isopropylalkohol und sec-Butylalkohol durch direkte katalytische Hydratisierung der entsprechenden Olefine mit Wasser in Gegenwart saurer Katalysatoren in einem Riesel- oder Sumpfreaktor bei erhöhter Temperatur und unter erhöhtem Druck und Rückführung des bei der Umsetzung als Nebenprodukt gebildeten und aus dem Umsetzungsprodukt isolierten Ethers, dadurch gekennzeichnet, daß der Ether separat von den Reaktanden 5 bis 30 % vor Ende der Reaktionsstrecke, bezogen auf die Gesamtlänge, in den Reaktor eingespeist wird.

2. Verfahren nach Anspruch 1, dadurch gekennzeichnet, daß der Ether 10 bis 20 % vor Ende der Reaktionsstrecke, bezogen auf die Gesamtlänge, in der Reaktor eingespeist wird.

### Claims

1. Process for the continuous preparation of isopropyl alcohol and sec-butyl alcohol by direct catalytic hydration of the corresponding olefins with water in the presence of acidic catalysts in a trickle or sump reactor at elevated temperature and pressure and by recycling of the ether formed as a byproduct during the reaction and isolated from the reaction product, characterized by feeding the ether separately from the reactants to the reactor at 5 to 30 % before the end of the reaction zone, relative to the total reactor length.

2. Process according to claim 1, characterized by feeding the ether to the reactor at 10 to 20 % before the end of the reaction zone, relative to the total reactor length.

### Revendications

1. Procédé de préparation continue d'alcool isopropylique et sec-butylique par hydratation catalytique directe des oléfines respectives avec de l'eau en présence de catalyseurs acides dans un réacteur à courant descendant ou à courant ascendant à une température et sous une pression élevées et avec recyclage de l'éther formé comme sous-produit au cours de la transformation et isolé du produit de transformation, caractérisé en ce que l'éther est introduit dans le réacteur séparément de la charge à un endroit situé entre 5 et 30 % avant la fin de la zone réactionnelle, par rapport à la longueur totale.

2. Procédé selon la revendication 1, caractérisé en ce que l'éther est introduit dans le réacteur à un endroit situé entre 10 et 20 % avant la fin de la zone réactionnelle, par rapport à la longueur totale.

# F i g.1

# F i g.2